# FASCICULE DE BREVET EUROPEEN

(11) **EP 3 740 118 B1**
(45) Date de publication et mention de la délivrance du brevet: **06.11.2024**
(21) Numéro de dépôt: 19703948.0
(22) Date de dépôt: 16.01.2019
(51) Int. Cl.: A61B 5/00, A61B 5/145, A61N 1/32, A61B 5/053

(54) **SYSTÈME DE SURVEILLANCE CORPORELLE PAR ÉLECTROPHORÈSE**
SYSTEM ZUR KÖRPERÜBERWACHUNG DURCH ELEKTROPHORESE
SYSTEM FOR BODY MONITORING BY ELECTROPHORESIS

(30) Priorité: 16.01.2018 FR 1850346
(43) Date de publication de la demande: 25.11.2020
(73) Titulaire: WIZP AS, 3183 Horten (NO)
(72) Inventeur: PIERART, Luc, 94800 VILLEJUIF (FR)
(74) Mandataire: Regimbeau
(86) Numéro de dépôt international: PCT/EP2019/051073
(87) Numéro de publication internationale: WO 2019/141742

(56) Documents cités:
- EP-A1- 1 183 066

## Description

### DOMAINE TECHNIQUE GENERAL

La présente invention concerne la micro-fluidique. Plus précisément, elle concerne un système de surveillance corporelle via analyse de liquide interstitiel.

### ETAT DE L'ART

Certaines pathologies comme le diabète nécessitent une surveillance quotidienne de paramètres biochimiques du corps humain, i.e. des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins d'un capteur analytique), de façon à estimer le ou les paramètres cible.

On connaît aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, c'est-à-dire sans nécessité de percer régulièrement la peau et de prélever.

En particulier, il a été proposé un dispositif porté au poignet appelé GlucoWatch, mettant en oeuvre un phénomène appelé iontophorèse (ou ionophorèse) dans lequel champ électrique permet « d'attirer » le liquide interstitiel à travers la peau jusqu'à un capteur sur la paroi du dispositif. Ce concept a cependant été abandonné rapidement car 6% seulement des patients supportaient la douleur d'extraction électrique. De surcroît les résultats des mesures étaient peu fiables.

Il a été proposé alternativement des sondes transcutanées prenant la forme d'un patch autocollant plaquant un « capteur-aiguille » juste sous la peau, de sorte à mettre le capteur en communication fluidique permanente avec le liquide interstitiel, pour une surveillance continue.

Certaines de ces sondes transcutanées de type patch comprennent des moyens de communications sans fil permettant de remonter les mesures sur le liquide interstitiel par exemple à un terminal mobile, pour stockage et traitement (vérification de seuils et de variations, réalisation de statistiques, déclenchement d'alertes si nécessaire, etc.). On citera par exemple les systèmes sugarBEAT^{™} ou FreeStyle Libre.

De tels systèmes apportent satisfaction (les patchs sont nettement plus fiables et moins douloureux que les dispositifs à ionophorèse), mais s'avèrent contraignants et couteux du fait du patch, à usage unique, qu'il faut changer régulièrement. De plus les patchs ont tendance à se décoller, s'arracher lorsque l'on change ses vêtements ou fait des mouvements brusques, et sont très visibles : le porteur peut être stigmatisé comme « malade ».

Il serait souhaitable de disposer d'un système électronique alternatif portatif qui évite le recours aux patchs, sans douleur, et sans qu'il y ait pour autant le moindre risque hygiénique.

On connaît d'autres systèmes, plus volumineux, qui ne peuvent se porter au poignet. Par exemple, la percussion de micro-aiguilles pleines à l'aide d'un percuteur sur la peau, puis la dépression à l'aide d'une chambre à vide ou la percussion de micro-aiguilles pleine puis extraction avec une chambre à vide. Il est en outre difficile d'avoir une aspiration continue. Le document EP1183066 divulgue un dispositif comprenant toutes les caractéristiques techniques du préambule de la revendication 1.

### PRESENTATION DE L'INVENTION

L'inconvénient des systèmes précités résident dans le fait qu'ils sont soit douloureux, soit trop volumineux pour être portés au poignet.

La présente invention se rapporte à un dispositif de surveillance corporelle selon la revendication 1.

Le dispositif peut comprendre les caractéristiques, prises seules ou en combinaison :
- au moins une aiguille fait office d'électrode d'électrophorèse, préférablement la totalité des aiguilles,
- l'autre électrode d'électrophorèse est une surface plane, comme une plaque,
- Le dispositif comprend au moins un capteur d'une grandeur physique de liquide interstitiel, le capteur étant configuré pour être au contact du liquide interstitiel afin de mesurer une grandeur physique du liquide interstitiel,
- le dispositif comprend un circuit fluidique comprenant l'au moins une aiguille et le capteur,
- l'au moins une aiguille forme des moyens de prélèvement transcutané par exemple sous la forme d'une ou d'une pluralité d'aiguilles creuses ou pleines,
- l'au moins un capteur est situé au niveau de l'extrémité de l'aiguille configurée pour être insérée dans le corps,
- l'au moins une aiguille est pleine,
- le dispositif comprend en outre au moins une aiguille, distincte de l'aiguille-capteur, faisant office d'électrode d'électrophorèse,
- les deux électrodes sont formées par des aiguilles,
- ladite aiguille-électrode comprend du métal entièrement ou sur sa surface extérieure seulement,
- une des aiguilles est plus courtes que les autres, ladite aiguille formant une électrode pour mesurer la conductivité afin de savoir si les aiguilles sont correctement insérées,
- une aiguille comprend deux électrodes, configurées pour mesurer la conductivité selon un plan transversal à une direction d'extension de l'aiguille afin de savoir si l'aiguille est emplie de liquide,
- le dispositif est configuré pour mesurer le pH de la peau,
- le pH de la peau est mesuré par au moins une des électrodes d'électrophorèse,
- au moins une des électrodes est une aiguille (préférablement une microaiguille) configurée pour être insérée dans la peau.

L'invention concerne aussi une capsule comprenant un dispositif tel que décrit précédem ment.

L'invention concerne aussi un système comprenant :
- un dispositif ou une capsule telle que décrite précédemment,
- des moyens de traitement de données configurés pour traiter les données du capteur, et
- un générateur de signaux électriques configurés pour alimenter l'ensemble d'électrodes d'électrophorèse, le générateur de signaux électriques est configuré pour générer des signaux inférieurs ou égaux à 300 µA.

Le système peut comprendre les caractéristiques suivantes, prises seules ou en combinaison:
- le système comprend un boitier et une capsule tel que décrit précédemment, configurée pour s'engager avec le boitier de façon amovible, le boitier comprenant les moyens de traitements de données et le générateur de signaux électriques,
- le système comprend un conductimètre configuré pour mesurer la mesure de conductivité de la peau à l'aide d'électrodes, préférablement les électrodes d'électrophorèse,
- le signal électrique généré par le générateur de signal électrique tient compte de la mesure de conductivité de la peau,
- le système comprend en outre un capteur d'hygrométrie, un capteur cardiaque et/ou un accéléromètre, pour mesurer la sueur de la peau et/ou corriger les mesures du capteur,
- le boitier comprend des moyens d'attache au corps réutilisables, configurés pour maintenir les aiguilles au contact de la peau.

L'invention concerne aussi un procédé de prélèvement ou mesure de liquide interstitiel, mis en oeuvre à l'aide d'un dispositif ou capsule ou système tel que décrit précédemment par exemple, comprenant une étape de :
- électrophorèse à l'aide d'électrodes pour accumuler du liquide interstitiel sous la peau à l'aide des électrodes sans que le liquide ne traverse la peau, où l'électrophorèse est appliquée à des valeurs inférieures ou égales à 300µA
- prélèvement ou mesure de liquide interstitiel accumulé à l'aide de moyens de prélèvement ou mesure transcutané (une ou des aiguilles par exemple).

Il est divulgué aussi une utilisation d'une électrophorèse sous-cutanée à l'aide de deux électrodes pour accumulé le liquide interstitiel et le prélever ou le mesurer par une aiguille positionnée au niveau de l'accumulation de liquide interstitiel.

### PRESENTATION DES FIGURES

D'autres caractéristiques et avantages de la présente invention apparaîtront à la lecture de la description qui va suivre d'un mode de réalisation préférentiel. Cette description sera donnée en référence aux dessins annexés dans lesquels :
- La figure 1 illustre un schéma représentant un système comprenant une capsule, un boitier et un patch disposé sur la capsule,
- Les figures 2a à 2c représentent des vues tridimensionnelles de l'engagement de la capsule dans le boitier,
- Les figures 3a à 3c représentent des vues tridimensionnelles de la capsule,
- La figure 4 représente une aiguille électrode,
- La figure 5 représente une pluralité d'aiguilles-capteurs, selon un mode de réalisation.

### DESCRIPTION DETAILLEE

### Architecture générale

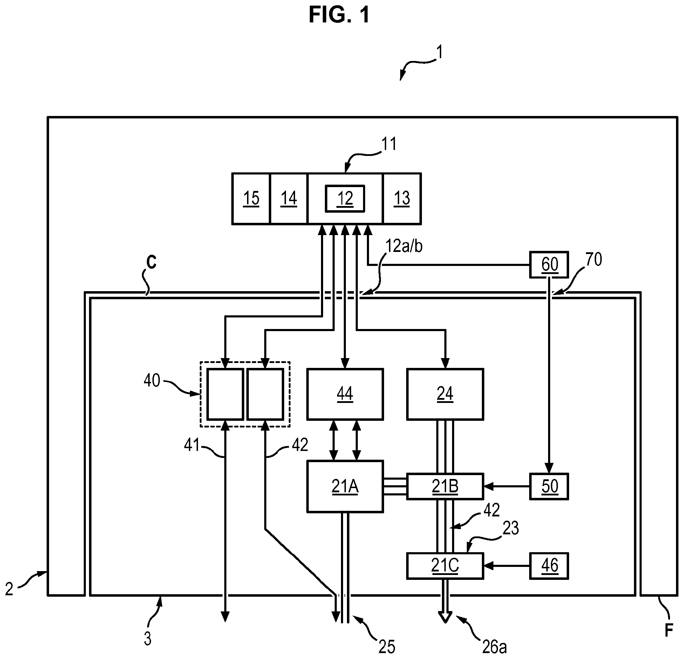

En référence à la **figure 1****,** qui en représente un schéma général, la présente invention concerne un système électronique 1 de surveillance corporelle.

Par surveillance corporelle, on entend la vérification de constantes biochimiques d'une personne porteuse du système 1, typiquement la concentration en une protéine, une hormone, un marqueur, en oxygène, en nutriments, etc., dans le liquide interstitiel de la personne. On citera l'exemple de la glycémie. L'homme du métier pourra surveiller si besoin d'autres grandeurs physiques corporelles telles que la température, l'hydratation, etc.

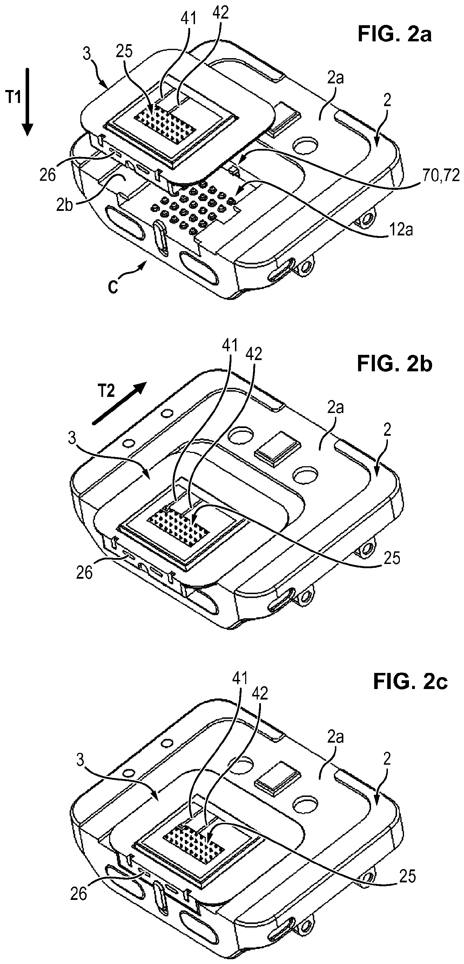

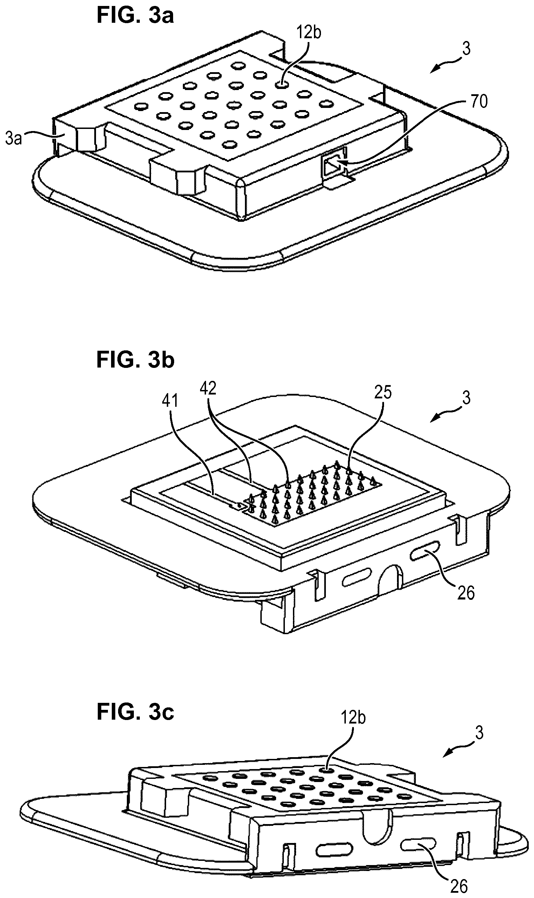

Comme cela sera explicité par la suite (voir **figures 2a à 2c**), le système peut être séparé en deux modules : un boitier 2 et une capsule 3 (voir **figures 3a à 3c**). On utilisera le terme de dispositif, avec la même référence 3, pour ce qui concerne la capsule.

Le dispositif 3 comprend ainsi au moins un capteur 24 d'une grandeur physique du liquide interstitiel, en particulier un capteur de nature « biochimique » c'est-à-dire permettant l'analyse du liquide interstitiel notamment en détectant un composé par exemple grâce à plusieurs électrodes au contact du liquide interstitiel, et avantageusement au moins un deuxième capteur tel qu'un capteur de température permettant d'ajuster l'analyse du liquide interstitiel prélevé (prise en compte de la viscosité du fluide en fonction de la température).

Il comprend également des moyens de traitement de données 11 (en particulier un processeur ou un microcontrôleur) configurés pour traiter des mesures acquises par le capteur 24, et le cas échéant des moyens de stockage de données 12 (notamment une mémoire, en particulier de type flash, et/ou la mémoire du microcontrôleur) permettant par exemple de stocker ces mesures, et/ou une date de la première utilisation de chaque capteur 24 pour calculer une date de péremption du ou des capteurs 24 (les capteurs biochimiques ont une durée de vie limitée). Les moyens de traitement de données 11 servent aussi à générer des consignes vers différentes composants. Dans le cadre de cette description, ces différentes fonctions sont assurées par une même unité. Toutefois, il est possible de prévoir des processeurs dédiés. Le système comprend également une batterie 13 pour l'alimentation électrique des composants, avantageusement rechargeable, par exemple via le port 10 (dont on comprend qu'il peut également servir à connecter le système 1 par exemple à un ordinateur pour télécharger les données acquises et/ou traitées).

De façon préférée le système 1 peut comprendre des moyens de connexion sans fil (en particulier de type WiFi, mais également Bluetooth, voire 3G/4G) pour connexion à un réseau, en particulier internet, et une interface utilisateur tel qu'un écran, éventuellement tactile pour afficher les résultats de la surveillance à l'utilisateur.

On comprendra que l'homme du métier connait des algorithmes de traitement de mesures de capteurs 24 et des interfaces associées, et saura les implémenter dans le présent système 1.

Enfin, le dispositif 3 comprend en outre un ensemble 40 d'électrodes d'électrophorèse, configurés pour être en contact avec la peau et provoquer une électrophorèse sous la peau, c'est-à-dire provoquer une concentration du liquide interstitiel. Pour alimenter cet ensemble d'électrode 40, le système comprend un générateur de signaux électriques 14. Etant donné les tensions et ampérage impliqués, ce générateur de signaux 40 électriques peut être confondus avec les moyens de traitement de données. Le générateur de signaux électriques 14 est piloté par les moyens de traitement de données 11.

### Premier mode de réalisation : circuit fluidique

Le présent dispositif 3 comprend un circuit fluidique 20 sur lequel sont disposés des moyens de prélèvement transcutané 25 de liquide interstitiel, ledit capteur 24, et une pompe, ladite pompe étant commandée par les moyens de traitement de données 11.

Lesdits moyens de prélèvement transcutané 25 de liquide interstitiel consistent avantageusement en un réseau de micro-aiguilles au contact de la peau lorsque le dispositif 3 est placé sur le corps d'une personne (en verra comment plus loin). Les micro-aiguilles permettent l'extraction de liquide interstitiel du derme de façon indolore sans perlement de sang.

De façon préférée, lesdits moyens de prélèvement transcutané 25 comprennent entre quatre et seize microaiguilles (en particulier quatre, neuf ou seize sous forme de carré), sensiblement pyramidales, avec des pointes d'une hauteur comprise entre 0.3mm et 0.8mm, chacune avec un trou d'un diamètre compris entre 0.04 et 0.22mm. Chacune de ces caractéristiques avantageuses des micro-aiguilles peut être prise séparément ou en combinaison avec les autres.

La pompe peut être réalisée sous différentes formes.

Sur la figure 1, la pompe comprend une mousse 21A ou 21B ou 21C ou plusieurs mousses 21A, 21B, 21C en série qui aspire le fluide grâce à leur hydrophilie et à la capillarité. Par exemple, la mousse 21A aspire une première fois le fluide, qui est ensuite aspiré depuis la mousse 21A par la mousse 21B, qui est ensuite aspiré depuis la mousse 21B par la mousse 21C. La mousse 21C est en contact avec le capteur 24, afin que ce dernier puisse effectuer des mesures sur le liquide interstitiel stockée dans la mousse 21B. La mousse 21C, dite mousse d'évacuation a pour fonction de vider la mousse 21b et de faciliter l'évaporation du liquide qu'elle stocke. Cette évaporation peut se faire naturellement, avec l'air ambiant, ou à l'aide d'un radiateur 46 qui vient chauffer ladite mousse 21b. Le radiateur 46 est alors piloté par les moyens de traitement de données 11. La circulation est alors à double sens, c'est-à-dire qu'un équilibre est effectué entre les mousses et le liquide interstitiel de la peau.

La mousse 21C est décrit plus en détail dans la demande de brevet français au nom du même demandeur déposée concomitamment à la présente demande, intitulée « Système de surveillance corporelle avec double mousse ». Les références numériques sont identiques

La mousse de mesure 21B peut être mobile, à l'aide d'un mécanisme passif 50 dans la capsule 3, attachée à la mousse 21B et un mécanisme actif 60, dans le boitier 2, et reliés entre eux par une interface 70, comme décrite dans la demande de brevet français au nom du même demandeur déposée | concomitamment à la présente demande, intitulée « Système de surveillance corporelle avec mousse mobile ». Les références numériques sont identiques.

Le système 1 peut aussi comprendre un conductimètre 44, qui permet de savoir si les mousses 21A ou 21B sont imbibées de liquide. En particulier on mesure la conductimétrie de la mousse mobile 21B (non illustré). Un système avec plusieurs mousses est décrit en détail dans la demande de brevet français au nom du même demandeur déposée concomitamment à la présente demande, intitulée « Système de surveillance corporelle avec double mousse ». Les références numériques sont identiques.

Alternativement, la pompe 21 peut être une micro-pompe piézoélectrique permettant l'aspiration grâce à un système de vibration membranaire. Lorsqu'elle fonctionne (sur commande des moyens de traitement de données 11) elle va provoquer l'extraction transcutanée (à travers l'épiderme) de liquide interstitiel depuis le corps (le derme sous l'épiderme), sa circulation à travers le circuit 20 jusqu'au capteur 24 qui va l'analyser, puis son arrivée au niveau de la mousse absorbante (non représentée) permettant ainsi de le stocker et de l'évacuer par évaporation (la mousse est avantageusement en échange avec l'atmosphère, en particulier à travers un tissu poreux aux gaz, de sorte à permettre cette évaporation, mais avantageusement étanche aux liquides (par exemple à base de polytétrafluoroéthylène) de sorte à éviter la pénétration de liquides comme la sueur depuis l'extérieur). La circulation est alors uniquement en sens unique, ce qui fait que les moyens de prélèvement 25 sont l'unique entrée de fluide, et la mousse est l'unique sortie. Il n'y a pas de cycle.

### Ensemble d'électrode d'électrophorèse

L'ensemble 40 d'électrodes comprend au moins deux électrodes 41, 42 (de type une électrode positive 41 et une électrode négative 42) qui permettent d'appliquer un champ électrique par une différence de tension. Sous l'influence du champ électrique, les ions Na+ présents dans le liquide interstitiel se déplacent vers l'électrode négative, entrainant avec eux les molécules de glucose, ce qui provoque une accumulation sous la peau (*via* une mini-inflammation).

Grâce à cette concentration, le prélèvement par les moyens de prélèvement transcutané 25 s'en trouvent simplifié.

En outre, comme l'électrophorèse n'est pas utilisée pour que le liquide interstitiel traverse la peau, l'électrophorèse peut être appliqué à des valeurs plus faible (intensité de 10µA à 300µA), ce qui évite les douleurs rédhibitoires existantes avec ce genre de technique.

Un exemple de signal d'électrophorèse comprend une tension de 23V, ±2 V, avec une fréquence de 200Hz.

L'ensemble 40 d'électrodes peut être composés d'aiguilles ou d'une surface plane telle qu'une plaque. L'aiguille pénètre dans la peau tandis que la plaque demeure sur la peau.

L'électrode négative 42 est positionnée à proximité des moyens de prélèvement 25 afin d'y augmenter la présence de liquide interstitiel et favoriser leur prélèvement. Par « à proximité », on signifie que les moyens de prélèvement 25 sont positionnés là où la concentration de liquide interstitiel augmente grâce aux électrodes, préférablement là où la concentration est maximale. Dans un mode de réalisation, les deux électrodes 41, 42 sont situées de part et d'autre des moyens de prélèvement 25.

Dans un mode de réalisation particulièrement avantageux décrit ci-dessous, les moyens de prélèvement 25 comprennent une pluralité de microaiguilles creuses. Une micro-aiguille désigne simplement une aiguille de petite taille (des dimensions telles que précitées précédemment).

Dans un mode de réalisation préférentiel, l'électrode négative 42 est une aiguille, positionnée avec la pluralité d'aiguilles des moyens de prélèvement 25. En particulier, cette électrode peut être une aiguille des moyens de prélèvement 25. Cela permet d'avoir un dispositif 3 plus compact, qui présente moins d'éléments à insérer dans la peau, et qui soit particulièrement efficace puisque le prélèvement se fait exactement au niveau de l'électrode négative.

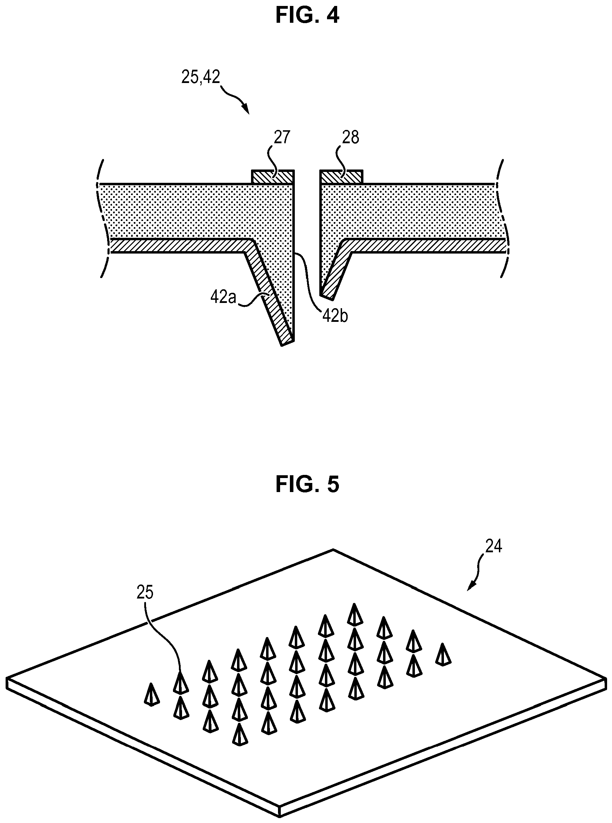

A cette fin, la **figure 4** illustre une telle aiguille-électrode 25, 42. Afin de fonctionner en électrode, l'aiguille (qui est un tube, creux) comprend du métal sur sa surface extérieure. Afin de fonctionner en moyens de prélèvement 25, l'aiguille-électrode 25, 42 comprend à l'intérieur un revêtement 42b adapté pour une micro-canule.

La présence de métal sur la surface extérieure se fait soit à l'aide d'une électrode en métal, auquel cas l'intérieur peut comprendre le revêtement 42b, soit à l'aide d'une électrode en polymère avec par exemple à l'aide d'un revêtement 42a en métal.

Dans une variante, plusieurs aiguille 25, voire la totalité des aiguilles 25 font office d'électrode.

En effet, comme illustré sur les **figures 2a à 2c**, 3b et 4, toutes les aiguilles 25 (leur revêtement externe plus exactement) sont reliées à la même électrode 42 typiquement par une plaque en métal, qui assure la continuité électrique.

### Ensemble d'électrode pour conductivité

Le système 1 peut aussi comprendre un conductimètre comprenant des électrodes pour mesurer la conductivité de la peau (qui change en fonction de l'humidité de la peau - la sueur, le temps extérieur par exemple). Le conductimètre comprend aussi un autre générateur 15 de signaux électriques, configurés pour alimenter les électrodes de conductivité. En pratique, ce générateur 15 est le même que le générateur de signaux électriques 14. Le générateur 15 de signaux électriques est piloté par les moyens de traitement de données 11.

Dans un mode de réalisation préférentiel illustré sur les figures, les électrodes de conductivité sont les mêmes que les électrodes d'électrophorèse 41, 42. Cela permet de gagner en compacité et en simplicité, mais aussi d'avoir une mesure de conductivité extrêmement pertinente pour l'électrophorèse.

La mesure de conductivité est gérée par les moyens de traitement de données 11. A partir de cette mesure, la valeur de l'électrophorèse est adaptée pour éviter d'utiliser une tension/intensité trop faible (donc pas de concentration) ou trop forte (donc des douleurs).

Le contrôle de la conductivité se fait avant ou concomitamment à l'utilisation de l'électrophorèse pour accumuler le liquide interstitiel pour le prélèvement ou mesure.

Grâce à l'obtention de la conductivité, on peut caractériser le corps humain et mieux adapter les valeurs utilisées pour procéder à la mesure du taux de glucose.

Un capteur d'hygrométrie, un capteur cardiague et/ou un accéléromètre, en particulier en combinaison avec la mesure de la conductivité de la peau, peuvent être utilisés pour évaluer le niveau de sueur sur la peau. La connaissance du niveau de sueur permet de mieux corriger les mesures, notamment pour la glycémie ou le lactate, car la sueur est chargée en glucose et lactate.

La mesure du pH de la peau est une donnée pertinente pour caractériser la sueur. Cette mesure peut être faite à l'aide d'électrode dans le dispositif, en particulier une des électrodes d'électrophorèse, notamment en mettant un revêtement particulier à un endroit spécifique de l'électrode. Les moyens de traitement de données sont alors programmés pour pouvoir déterminer le niveau de sueur à partir de la mesure de pH.

En utilisant les deux techniques (conductivité d'une part, pH de l'autre), on peut obtenir une donnée relative à la sueur plus précise.

Une autre mesure de conductivité peut être faite pour savoir si les aiguilles sont bien enfoncées dans la peau ou non. A cette fin, la pluralité d'aiguilles 25 comprend au moins une aiguille de longueur inférieure (c'est-à-dire que la pointe n'arrive pas au même niveau). Cette aiguille plus courte est reliée à une électrode et une aiguille de longueur normale est reliée à une autre électrode. En mesurant la conductivité entre ces deux électrodes, on peut savoir si les aiguilles sont bien insérées ou non pour prélever du liquide interstitiel.

On définit de la même façon un conductimètre comprenant l'aiguille plus courte précitée et une autre aiguille, ainsi qu'un générateur de signaux électrique, qui est généralement le générateur 14.

### Mesure de la conductivité transversale des aiguilles

Afin de vérifier que les aiguilles 25 prélèvent bien du liquide interstitiel, une aiguille parmi la pluralité d'aiguilles 25 peut être associé à un conductimètre comprenant des électrodes, comme illustré sur la **figure 4** (ce principe est applicable à toute les aiguilles). En particulier, deux électrodes 27, 28 sont disposés de part et d'autre de l'aiguille 25, dans un plan de section orthogonale à l'axe d'extension principale de l'aiguille.

Les électrodes peuvent être formées par des éléments spécifiques, par exemple au niveau d'une extrémité de l'aiguille 25 (extrémité opposée à l'extrémité qui prélève le liquide interstitiel), comme représenté sur la **figure 4****.**

En reliant les électrodes à un conductimètre, on peut connaitre la valeur de la conductivité de l'aiguille. Cette conductivité dépend de la présence ou non de liquide interstitiel dans l'aiguille 25. En présence de ce dernier, le conductimètre indiquera une valeur plus importante.

Le conductimètre comprend en outre un générateur de signaux électriques, typiquement le générateur 15. Le générateur de signaux électriques est piloté par les moyens de traitement de données 11.

Pour des raisons évidentes de praticité, il s'agit du générateur de signaux électriques 14. La donnée de conductimétrie est ensuite traitée par les moyens de traitement de données 11.

### Mesure de la conductivité globale des aiguilles

Afin de connaitre le niveau global de remplissage des aiguilles considérées comme un tout, il est possible de mesurer la conductivité globale, par exemple en mettant une électrode d'un côté du groupe d'aiguilles et une électrode de l'autre côté du groupe d'aiguilles.

### Capsule

Contrairement aux systèmes connus, le système 1 est intégral. Cela signifie qu'il n'y a pas un module principal tel qu'un terminal mobile connecté sans fil à un module secondaire encombrant et couteux (du fait de la nécessité de l'équiper d'une batterie, de moyens de communication sans fil, etc.) prenant la forme d'un patch collé à la peau. Le système 1 est ainsi autonome.

Pour être utilisé, il est soit plaqué sur la peau au besoin par l'utilisateur pendant quelques secondes (il est dans ce cas dépourvu de tout moyen d'attache au corps), soit directement porté sur le corps, en particulier sur un membre, et préférentiellement au poignet. Le dispositif 3 prend alors par exemple la forme d'une montre, avec une face F contre la peau du bras, et une face opposée accueillant par exemple un écran.

A ce titre, il comprend avantageusement des moyens d'attache au corps réutilisables, consistant typiquement en une sangle ou un bracelet configuré pour entourer le membre (et non un élément collant), en particulier un bracelet-montre. « Réutilisable » s'entend ici en effet par opposition à « à usage unique » comme c'était le cas des patchs, qui ne peuvent être réutilisés après avoir été décollé de la peau et doivent être jetés. Un bracelet peut être ouvert et refermé de nombreuses fois.

Le système 1 est configuré pour que lorsqu'il est placé sur la peau (i.e. lorsque les moyens d'attache le fixent au corps) les moyens de prélèvement transcutané 25 soient maintenus contre la peau (ou du moins au voisinage immédiat) pour permettre le prélèvement.

Dans tous les cas, le système 1 contient deux sous-ensembles :
- un boitier 2 dans lequel sont disposés au moins les moyens de traitement de données 11 (ainsi que les composants principaux tel que la batterie, la mémoire, l'éventuelle interface utilisateur, etc.) ;
- une capsule 3 dans laquelle sont disposés le capteur 24, l'ensemble d'électrodes 40, la pompe 21 lorsqu'il s'agit d'une mousse par exemple, la mousse 21A, B ou C (comme expliqué préférentiellement en échange avec l'atmosphère pour favoriser l'évaporation du fluide et ainsi limiter sa stagnation, le cas échéant via une fenêtre 26 à travers la capsule 3 - voir **figures 3b et 3c**) et les moyens de prélèvement transcutané 25, la capsule 3 étant configurée pour s'engager avec le boitier 2 de façon amovible. La capsule 3 s'engage de façon préférée dans une cavité C du boitier 2 située sur sa face F destinée à être en contact de la peau.

Dans la présente description, le terme de dispositif 3 se rapporte à la capsule.

La capsule comprend donc une face destinée à être posée sur une surface sensiblement plane, comme le poignet d'une main. Sur cette face se trouvent donc les électrodes 41, 42 et les aiguilles 25.

Formulé autrement, et comme l'on voit sur la figure 1 et les **figures 2a-****2c** qui représentent un mode de réalisation préféré, le système 1 reste composé de deux modules 2, 3 mais ces derniers ne sont pas séparés physiquement comme ce pouvait être le cas dans l'art antérieur et sont même en connexion directe, mécanique.

Le boitier 2 et la capsule 3 comprennent pour cela moyens de transmission pour transmettre des signaux électriques, tels que des liaisons sans fil (émetteur/récepteur) sans contact ou des des connectiques électriques 12a, 12b (des contacts, voire **figures 2a****,** **3a**) et, dans le cas d'une pompe dans le boitier, des connectiques fluidiques non illustrée). Les connectiques électriques 12a, 12b sont configurées de sorte que, lorsque la capsule 3 est engagée avec le boitier 2, lesdites connectiques électriques 12a, 12b assurent une connexion entre d'une part les moyens de traitement de données 11, le ou les générateurs de signaux électriques 14, 15 et d'autre part le capteur 24 (pour remonter les données de mesures) et l'ensemble d'électrodes d'électrophorèse 40 (et les autres composants qui nécessitent des consignes et/ou d'envoyer des signaux), et les électrodes de conductivité 27, 28. Lorsqu'elles sont nécessaires, lesdites connectiques fluidiques 22a, 22b, 22c, 22d assurent que le circuit fluidique 10 s'étend de manière étanche à la fois dans le boitier 2 et la capsule 3 (de sorte que la puissance d'aspiration de la pompe 21 ne soit pas altérée).

La capsule 3 constitue ainsi un sous-ensemble interchangeable du système 1 qui peut être choisi selon le type de surveillance voulu. En effet, dans la mesure où la capsule contient le ou les capteurs 24, changer de capsule permet de changer de capteurs 24 si ceux-ci sont en fin de vie ou si l'on souhaite changer de grandeur physique mesurée, en une manipulation simple, rapide et sure, sans devoir jeter d'autres parties (en particulier le boiter 2). On note à ce titre que la capsule 3 préserve très efficacement les capteurs 24, et a besoin d'être changée moins souvent qu'un patch (la même capsule peut être utilisée un mois).

Et dans la mesure où la capsule 3 ne contient ni pompe, ni équipement électronique avancé tel qu'une batterie ou des moyens de communication sans fil, elle est nettement moins chère qu'un patch.

Le présent système 1 est donc bien plus pratique que les systèmes connus de l'art antérieur, mais surtout beaucoup moins cher à l'usage, sans qu'il y ait le moindre risque hygiénique, et sans douleur due à l'insertion d'une aiguille ou à de l'électricité forte.

Dans un mode de réalisation avantageux (celui où la pompe est dans la capsule), la capsule 3 est la seule partie du système 1 qui peut être en contact de liquide interstitiel, puisqu'elle contient la mousse absorbante 21A, B ou C (au-delà de laquelle le fluide prélevé ne peut pas remonter). Ainsi, la capsule amovible empêche la contamination du boitier 2 et permet de pouvoir remplacer la capsule 3 en fin de vie des capteurs 24 ou changer de type de capsule 3 en une manipulation simple, rapide et sure, sans devoir jeter d'autres parties.

### Changement de capsule

La capsule 3 s'engage avec le boitier 2 selon deux mouvements de translation successifs T1 et T2 **(****figures 2a à 2c****).** Le mouvement inverse permet de désengager la capsule 3. Le premier mouvement T1 consiste à amener la capsule 3 en direction du boitier 2 pour attacher les connectiques électriques 12a/b et le deuxième mouvement T2 consiste à faire glisser la capsule 3 le long du boitier 2 pour engager la liaison mécanique 70. Les connectiques électriques 12a/b sont souples (avec ressort par exemple).

Grâce à des butées 3a **(****figure 3a****),** une fois la translation T2 effectuée, la capsule 2 est bloquée selon la direction de la translation T1. Ces butées 3a se coincent entre le fond de la cavité C et un rail 2b **(****figure 2a****)**

### Autres architectures générales

Dans un autre mode de réalisation, le dispositif est similaire à ce qui a été décrit précédemment, sauf qu'il ne comprend pas de capteur et sert simplement à prélever le fluide. Le fluide est ensuite récupéré (soit par exemple depuis les mousses) pour être analysé ailleurs.

Dans un autre mode de réalisation, le dispositif comprend au moins une aiguille (préférablement une pluralité), qui agissent comme des moyens de transpercement de la peau. Sur la ou les aiguilles sont positionnées un ou des capteurs, typiquement sous forme de revêtement (capteur chimique) au niveau de l'extrémité de l'aiguille qui s'insère dans le corps.

Dans ce mode de réalisation, il n'y a donc pas de prélèvement transcutané de fluide. La mesure obtenue par le capteur est ensuite récupérée puis traitée. La **figure 5** illustre de telles aiguilles-capteurs. Le document de Yoon et al. (« Fabrication of a Microneedle/CNT Hierarchical Micro/Nano Surface Electrochemical Sensor and Its In-Vitro Glucose Sensing Characterization", in Sensors 2013, 13(12), 16672-16681; doi:10.3390/s131216672) décrit un type de capteur-aiguille.

Il est possible de prévoir que certaines aiguilles servent à la mesure (et comprennent un capteur) et que certaines aiguilles servent uniquement à l'électrophorèse (notamment pour des questions de matériau et de revêtement).

Les aiguilles sont alors généralement pleines, mais elles peuvent aussi être creuses.

Mis à part le circuit fluidique et les éléments qui le forment qui ne sont plus présent, le reste du système de mesure 1 est applicable sur ce mode de réalisation (moyens de traitement, connectivité, etc.).

Ainsi, les principes décrits précédemment pour l'électrophorèse sont identiques, sauf que l'aiguille permet directement la mesure, sans prélèvement.

### Procédé

Le système décrit précédemment permet de mettre en oeuvre un procédé d'extraction de liquide interstitiel d'une façon efficace, très peu invasive et quasi-indolore.

Un tel procédé comprend :
- une étape d'application d'un champ électrique à l'aide des électrodes (deux électrodes, négative et positive), pour accumuler du liquide interstitiel près de l'électrode négative par électrophorèse, et,
- une étape de prélèvement ou mesure du liquide interstitiel accumulé à l'aide des moyens de prélèvement ou mesure transcutanés (typiquement des aiguilles) au niveau des électrodes.

L'étape d'électrophorèse n'est pas calibrée pour que le liquide interstitiel traverse la peau. Par conséquent, ce procédé permet d'utiliser un ampérage comprend entre 10 et 300µA, qui sont plus faible que les ampérages pour une électrophorèse devant permettre la traversée des molécules d'intérêt au travers de la peau.

Plus généralement, ce procédé est applicable à toute membrane séparant un liquide comprenant des ions ou éléments chargés situés derrière une membrane d'un autre environnement et pour lequel on souhaite extraire ce liquide au travers de la membrane.

### Utilisation

Le système et le procédé décrits précédemment relève plus généralement de l'utilisation d'une électrophorèse sous-cutanée à l'aide de deux électrodes pour concentrer le liquide interstitiel et le prélever par une aiguille.

Plus généralement, cette utilisation est applicable à toute membrane séparant un liquide comprenant des ions ou éléments chargés situés derrière une membrane d'un autre environnement et pour lequel on souhaite extraire ce liquide au travers de la membrane.

Grâce au système ou au procédé ou à l'utilisation présenté, il est possible de prélever plus de 4µl de liquide interstitiel sans provoquer des douleurs rédhibitoires chez l'utilisateur.

## Revendications

1. Dispositif (3) de surveillance corporelle, comprenant au moins une aiguille (25) configurée pour venir au contact du liquide interstitiel, l'aiguille étant configurée pour prélever ou mesurer une grandeur physique du liquide interstitiel,
**caractérisé en ce que**
le dispositif (3) comprend des électrodes (41, 42) d'électrophorèse configurées pour être en contact avec la peau et provoquer une électrophorèse sous la peau sans que le liquide interstitiel ne traverse la peau lorsqu'elles sont alimentées par un courant d'intensité inférieur à 300 µA, de préférence compris entre 10 µA et 300 µA,
et **en ce**
**que** l'au moins une aiguille (25) est positionnée au voisinage d'au moins une des électrodes (42),
de sorte que l'électrophorèse provoque une accumulation de liquide interstitiel au niveau de ladite au moins une des électrodes (42) en contact avec la peau et au niveau de l'aiguille (25) sans qu'il ne traverse la peau.

2. Dispositif (3) selon la revendication 1, dans lequel au moins une aiguille (25) fait office d'électrode d'électrophorèse (42), préférablement la totalité des aiguilles (25).

3. Dispositif (3) selon la revendication 2, dans lequel l'autre électrode d'électrophorèse (41) est une surface plane, comme une plaque.

4. Dispositif (3) selon l'une quelconque des revendications précédentes, comprenant en outre au moins un capteur (24) d'une grandeur physique de liquide interstitiel, le capteur étant configuré pour être au contact du liquide interstitiel afin de mesurer une grandeur physique du liquide interstitiel.

5. Dispositif (3) selon l'une quelconque des revendications 1 à 4, dans lequel l'au moins une aiguille forme des moyens de prélèvement transcutané (25) par exemple sous la forme d'une ou une pluralité d'aiguilles creuses.

6. Dispositif (3) selon la revendication 4, dans lequel l'au moins un capteur (24) est situé au niveau de l'extrémité de l'aiguille configurée pour être insérée dans le corps, le capteur (24) étant formé par revêtement, et l'aiguille-capteur est préférablement pleine.

7. Dispositif selon la revendication 6, comprenant en outre au moins une aiguille, distincte de l'aiguille-capteur, faisant office d'électrode d'électrophorèse (42).

8. Dispositif (3) selon l'une quelconque des revendications 1 à 7 en combinaison avec la revendication 2, dans lequel ladite aiguille-électrode comprend du métal (42a), entièrement ou sur sa surface extérieure seulement.

9. Dispositif (3) selon l'une quelconque des revendications 1 à 8, dans lequel une des aiguilles (25) est plus courtes que les autres, ladite aiguille formant une électrode pour mesurer la conductivité afin de savoir si les aiguilles sont correctement insérées.

10. Dispositif (3) selon l'une quelconque des revendications 1 à 9, dans lequel une aiguille (25) comprend deux électrodes (27, 28), configurées pour mesurer la conductivité selon un plan transversal à une direction d'extension de l'aiguille afin de savoir si l'aiguille est en contact ou remplie avec du liquide.

11. Dispositif (3) selon l'une quelconque des revendications 1 à 10, configuré pour mesurer le pH de la peau, par exemple à l'aide d'au moins une des électrodes d'électrophorèse (27, 28) afin de mesurer le niveau de sueur.

12. Dispositif (3) selon l'une quelconque des revendications 1 à 11, dans lequel au moins une des électrodes est une aiguille.

13. Capsule (3) comprenant un dispositif selon l'une quelconque des revendications 1 à 12.

14. Système (1) comprenant :
- un dispositif (2) selon l'une quelconque des revendications 1 à 12 ou une capsule selon la revendication 13,
- des moyens de traitement de données (11) configurés pour traiter les données du capteur (24), et
- un générateur (14) de signaux électriques configurés pour alimenter l'ensemble d'électrodes d'électrophorèse, le générateur (14) de signaux électriques est configuré pour générer des signaux inférieurs ou égaux à 300 µA.

15. Procédé de prélèvement ou mesure de liquide interstitiel, mis en œuvre à l'aide d'un dispositif selon l'une quelconque des revendications 1 à 12, ou d'une capsule selon la revendication 13 ou d'un système (1) selon la revendication 14, comprenant une étape de :
- électrophorèse à l'aide d'électrodes (41, 42) pour accumuler du liquide interstitiel sous la peau à l'aide des électrodes sans que le liquide ne traverse la peau, où l'électrophorèse est appliquée à des valeurs inférieures ou égales à 300µA,
- prélèvement ou mesure de liquide interstitiel accumulé à l'aide d'une aiguille (25).

## Patentansprüche

1. Vorrichtung (3) zur Körperüberwachung, umfassend mindestens eine Nadel (25), die ausgelegt ist, um mit der Interstitialflüssigkeit in Kontakt zu kommen, wobei die Nadel ausgelegt ist, um Interstitialflüssigkeit zu entnehmen oder eine physikalische Größe davon zu messen,
**dadurch gekennzeichnet, dass**
die Vorrichtung (3) Elektrophorese-Elektroden (41, 42) umfasst, die ausgelegt sind, um mit der Haut in Kontakt zu sein und eine Elektrophorese unter der Haut zu bewirken, ohne dass die Interstitialflüssigkeit die Haut durchquert, wenn sie von einem Strom mit einer Stärke von unter 300 µA, vorzugsweise von zwischen 10 µA und 300 µA, versorgt werden,
und dadurch, dass
die mindestens eine Nadel (25) in der Nähe von mindestens einer der Elektroden (42) positioniert ist,
so dass die Elektrophorese eine Ansammlung von Interstitialflüssigkeit im Bereich der mindestens einen der Elektroden (42) im Kontakt mit der Haut und im Bereich der Nadel (25) bewirkt, ohne dass sie die Haut durchquert.

2. Vorrichtung (3) nach Anspruch 1, wobei mindestens eine Nadel (25) als Elektrophorese-Elektrode (42) fungiert, vorzugsweise alle Nadeln (25).

3. Vorrichtung (3) nach Anspruch 2, wobei die andere Elektrophorese-Elektrode (41) eine ebene Fläche wie eine Platte ist.

4. Vorrichtung (3) nach einem der vorhergehenden Ansprüche, ferner umfassend mindestens einen Sensor (24) für eine physikalische Größe der Interstitialflüssigkeit, wobei der Sensor ausgelegt ist, um mit der Interstitialflüssigkeit in Kontakt zu sein, um eine physikalische Größe der Interstitialflüssigkeit zu messen.

5. Vorrichtung (3) nach einem der Ansprüche 1 bis 4, wobei die mindestens eine Nadel transkutane Probenahmemittel (25) beispielsweise in Form einer oder mehrerer hohler Nadeln bildet.

6. Vorrichtung (3) nach Anspruch 4, wobei sich der mindestens eine Sensor (24) im Bereich des Endes der Nadel befindet, das ausgelegt ist, um in den Körper eingeführt zu werden, wobei der Sensor (24) durch Beschichtung gebildet ist und die Sensornadel vorzugsweise massiv ist.

7. Vorrichtung nach Anspruch 6, umfassend ferner mindestens eine Nadel, die sich von der Sensornadel unterscheidet und als Elektrophorese-Elektrode (42) dient.

8. Vorrichtung (3) nach einem der Ansprüche 1 bis 7 in Kombination mit Anspruch 2, wobei die Elektrodennadel ganz oder nur an ihrer Außenfläche Metall (42a) umfasst.

9. Vorrichtung (3) nach einem der Ansprüche 1 bis 8, wobei eine der Nadeln (25) kürzer ist als die anderen, wobei die Nadel eine Elektrode zur Messung der Leitfähigkeit bildet, um festzustellen, ob die Nadeln richtig eingeführt sind.

10. Vorrichtung (3) nach einem der Ansprüche 1 bis 9, wobei eine Nadel (25) zwei Elektroden (27, 28) umfasst, die ausgelegt sind, um die Leitfähigkeit in einer Ebene quer zu einer Erstreckungsrichtung der Nadel zu messen, um zu ermitteln, ob die Nadel mit Flüssigkeit in Kontakt steht oder damit gefüllt ist.

11. Vorrichtung (3) nach einem der Ansprüche 1 bis 10, die ausgelegt ist, um den pH-Wert der Haut zu messen, beispielsweise mit Hilfe von mindestens einer der Elektrophorese-Elektroden (27, 28), um den Schweißpegel zu messen.

12. Vorrichtung (3) nach einem der Ansprüche 1 bis 11, wobei mindestens eine der Elektroden eine Nadel ist.

13. Kapsel (3), umfassend eine Vorrichtung nach einem der Ansprüche 1 bis 12.

14. System (1), umfassend:
- eine Vorrichtung (2) nach einem der Ansprüche 1 bis 12 oder eine Kapsel nach Anspruch 13,
- Datenverarbeitungsmittel (11), die ausgelegt ist, um die Daten des Sensors (24) zu verarbeiten, und
- einen Generator (14) elektrischer Signale, der ausgelegt ist, um die Elektrophorese-Elektrodenanordnung zu versorgen, wobei der Generator (14) elektrischer Signale ausgelegt ist, um Signale von unter oder gleich 300 µA zu erzeugen.

15. Verfahren zur Entnahme oder Messung von Interstitialflüssigkeit, das mit Hilfe einer Vorrichtung nach einem der Ansprüche 1 bis 12 oder einer Kapsel nach Anspruch 13 oder einem System (1) nach Anspruch 14 durchgeführt wird, umfassend einen Schritt der:
- Elektrophorese mit Hilfe von Elektroden (41, 42), um Interstitialflüssigkeit unter der Haut mit Hilfe der Elektroden anzusammeln, ohne dass die Flüssigkeit die Haut durchquert, wobei die Elektrophorese mit Werten von unter oder gleich 300 µA angewendet wird,
- Entnahme oder Messung von angesammelter Interstitialflüssigkeit mit Hilfe einer Nadel (25).

## Claims

1. Body monitoring device (3), comprising at least one needle (25) configured to come into contact with interstitial fluid, the needle being configured to sample or measure a physical quantity of the interstitial fluid,
**characterised in that**
the device (3) comprises electrophoresis electrodes (41, 42) configured to be in contact with the skin and cause electrophoresis under the skin without the interstitial liquid passing through the skin when they are supplied with a current of less than 300 µA, preferably between 10 µA and 300 µA,
and **in that**
that the at least one needle (25) is positioned in the vicinity of at least one of the electrodes (42),
so that electrophoresis causes an accumulation of interstitial liquid at said at least one of the electrodes (42) in contact with the skin and at the needle (25) without it passing through the skin.

2. The device (3) according to claim 1, wherein one needle (25) acts as an electrophoresis electrode (42), preferably all the needles (25).

3. The device (3) of claim 2, wherein the other electrophoresis electrode (41) is a flat surface, such as a plate.

4. The device (3) according to any one of the preceding claims, further comprising at least one sensor (24) of a physical quantity of interstitial liquid, the sensor being configured to be in contact with the interstitial liquid in order to measure a physical quantity of the interstitial liquid.

5. The device (3) according to any one of claims 1 to 4, in which the at least one needle forms transcutaneous sampling means (25) for example in the form of one or a plurality of hollow needles.

6. The device (3) of claim 4, wherein the at least one sensor (24) is located at the end of the needle configured for insertion into the body, the sensor (24) being formed by coating, and the sensor needle is preferably solid.

7. The device according to claim 6, further comprising at least one needle, distinct from the sensor needle, acting as an electrophoresis electrode (42).

8. The device (3) according to any one of claims 1 to 7 in combination with claim 2, wherein said electrode needle comprises metal (42a), wholly or on its outer surface only.

9. The device (3) according to any one of claims 1 to 8, in which one of the needles (25) is shorter than the others, said needle forming an electrode for measuring conductivity in order to know whether the needles are correctly inserted.

10. The device (3) according to any one of claims 1 to 9, wherein a needle (25) comprises two electrodes (27, 28), configured to measure conductivity in a plane transverse to a direction of extension of the needle in order to know whether the needle is in contact or filled with liquid.

11. The device (3) according to any one of claims 1 to 10, configured to measure the pH of the skin, for example using at least one of the electrophoresis electrodes (27, 28) in order to measure the sweat level.

12. The device (3) according to any one of claims 1 to 11, wherein at least one of the electrodes is a needle.

13. Capsule (3) comprising a device according to any one of claims 1 to 12.

14. System (1) comprising:
- a device (2) according to any one of claims 1 to 12 or a capsule according to claim 13,
- data processing means (11) configured to process the data from the sensor (24), and
- an electrical signal generator (14) configured to power the electrophoresis electrode assembly, the electrical signal generator (14) is configured to generate signals less than or equal to 300 µA.

15. Method for sampling or measuring interstitial liquid, implemented by device according to any one of claims 1 to 12, or a capsule according to claim 13 or a system (1) according to claim 14, comprising a step of :
- electrophoresis using electrodes (41, 42) to accumulate interstitial liquid under the skin using the electrodes without the liquid passing through the skin where electrophoresis is applied at values less than or equal to 300 µA,
- sampling or measuring of accumulated interstitial fluid using a needle (25).
